# EUROPEAN PATENT SPECIFICATION

(11) **EP 2 478 769 B1**
(45) Date of publication and mention of the grant of the patent: **24.06.2020**
(21) Application number: 10816501.0
(22) Date of filing: 17.09.2010
(51) Int. Cl.: A23J 1/00, A21D 2/36, A21D 2/38, A21D 6/00, A21D 13/04, A23K 10/12, A23K 10/16, A23K 10/30, A23L 5/00, A23L 7/104, A23L 31/00, A23L 7/00, C12N 1/14

(54) **PROCESS FOR PRODUCING FLOURS FROM FUNGUS MYCELIATED GRAIN**
VERFAHREN ZUR HERSTELLUNG VON MEHLEN AUS DEM PILZ MYZELIERTEM KORN
METHODE DE PRODUCTION DE FARINES AVEC DES GRAINES COLONISÉES PAR LE MYCÉLIUM DE CHAMPIGNONS

(30) Priority: 17.09.2009 BR PI0903764
(43) Date of publication of application: 25.07.2012
(73) Proprietor: Blazei Brazil LTDA, 70773-510 - Brasília D.F. (BR); Empresa Brasileira De Pesquisa Agropecuária Embrapa, 70770-917 Brasília - DF (BR); Fundação De Apoio À Pesquisa Do Distrito Federal -, 71205-060 - Brasília - DF (BR)
(72) Inventor: BEZERRA DE OLIVEIRA, Haroldo César, 70365-110 - Brasília - DF (BR); BRITTO DE OLIVEIRA, Soraia Cristina, 70365-110 - Brasília - DF (BR); PINHO SANTOS, John Kennedy, 71805-520 - Brasília DF (BR)
(74) Representative: Vossius & Partner Patentanwälte Rechtsanwälte mbB
(86) International application number: PCT/BR2010/000302
(87) International publication number: WO 2011/032244

(56) References cited:
- WO-A1-03/045161
- WO-A1-2006/016701
- WO-A2-2008/068602
- BR-A- PI0 603 003
- CN-A- 1 294 871
- CN-A- 1 739 361
- CN-A- 101 095 419
- JP-A- H10 127 273
- JP-A- 2003 093 020

## Description

### FIELD OF THE INVENTION

The present invention relates to a method for making flour having grains myceliated with different fungi, preferably basidiomycetes and ascomycetes. This process adds more nutritive value to flours, making them rich in several components of the human and animal diet, thereby reducing the cost of manufacturing and the time spent with the process as compared with current processes. These flours may be used for domestic and industrial preparation/manufacture of food such as vitamins, porridges, yogurts, soups, breads, biscuits, cakes, doughs, energy bars, cereals, fodder, and the like. The method can also be used to obtain active ingredients (ergosterol, beta glucan, linoleic and oleic acid, lectins, and the like), enzymes, proteins, amino acids, vitamins, mineral salts and the like for use in the chemical, foodstuff, cosmetics, phytotherapeutic, pharmaceuticals, textiles, paper and medicine industries. The invention is defined by the appended set of claims and any other aspects or embodiments set forth herein not falling within the scope of the claims are for information only.

### STATE OF THE ART

Food products made from microorganisms, such as yoghurt, red rice, wine, cheese, fermented beverages and the like and the use of proteins, vitamins and mineral salts-enriched flours are part of the daily diet of many people in different cultures. Flours are also widely used for the preparation of certain food or feed products. If not processed properly, flours prepared from grains which are subjected to a period of time for the microorganisms to grow may suffer physical, physicochemical and biological modifications which make it impossible for them to be used for the preparation of doughs and other traditional food products.

Flours traditionally used in the market have low protein content and/or are free of certain amino acids, vitamins and mineral salts, which are essential factors in the human and animal diet, being sometimes compensated by the addition during the manufacture process of some chemical and natural components which considerably increase the cost of the end product. The mixture of grain flour (wheat, sorghum and the like) with the powder of the fruiting body of a fungus results in a nutritionally-enriched product because of supplementation, but the costs are high due to the high prices for dried fungus in the market. Therefore, addition of fungi to basic foods such as pasta, biscuits, breads, energy bars and the like has been considered expensive. Also, fungi having high protein and vitamin levels are not used in feeding due to the rigidity of their fruiting bodies, as is the case of *Ganoderma lucidum,* or for having a strong flavor, for example, *Agaricus blazei.* Another issue of adding fungi to grains is the pest management needed for preventing other undesirable microorganisms from contaminating the grains.

The flours described herein are produced from grains myceliated with different and specific types of fungi, namely, basidiomycetes and ascomycetes, having known nutritional and medicinal properties, either in admixture or not, so as to provide a product having high nutritional value, but having lower production costs than the currently existing methods where the fungal fruiting body is added to flours. In addition, the process set forth herein demands less time, since the fungus does not need to be cultivated for the process to be executed. Fungus production cycle lasts four to six months until the beginning of harvest, while in the process for the production of flour having myceliated grains this time is reduced to 20 to 30 days and the yield per area is also higher.

The present document discloses the process for the production of flours from grain myceliated with different macroscopic fungi for use as a base or for direct use in the human and animal food preparation as well as for extraction of active ingredients (ergosterol, beta glucan, linoleic and oleic acid, lectins and the like), enzymes, proteins, amino acids, vitamins, mineral salts and the like, for use in the chemical, foodstuff, cosmetics, phytotherapeutic, pharmaceuticals, textile, paper, animal medicines and fodder industries.

Since the mycelium of the fungus grows directly in the grain breaking down fibers, secreting enzymes and exopolysaccharides and enriching the material with proteins, vitamins and mineral salts, the flour prepared therefrom provides a novel nutritionally-improved natural product with reduced costs as compared with the ingestion of the fungal fruiting body alone. Another important aspect to be considered in this technology is the reduction in the time needed for the mycelium to grow in the grain. In the usual procedures, the fungus production cycle lasts 4 to 6 months until the beginning of harvest and it may be even longer. In the process of producing flour from myceliated grains of the present invention, this period of time is reduced to 20 to 30 days, the yield per area, that is, mycelial growth, being often higher than that of other described methods.

The myceliated grain flour can be prepared from different grains, either germinated (sprouts) or not, preferably: wheat, triticale, sorghum, corn, maize, amaranth, coffee, sesame, linseed, quinoa, rye, rice, sunflower, peanut, pea, lentil, chickpea, millet, oat and soy, either in admixture or not, myceliated with different edible and/or medicinal fungus species. The fungus used in the process may comprise, preferably: *Pleurotus ostreatus, P. sajor-caju, P. ostreatoroseus, P. eringii, Ganoderma lucidum, G. Applanatum, Ganoderma tsugae, Flammulina velutipes, Lentinula edodes, Lentinus strigellus, Morchella esculenta, M. conica, Macrolepiota procera, Volvariella volvacea, Grifola frondosa, Agaricus bisporus, A. blazei* or *A. brasiliensis, A. bitorques, A. brunnensis, Armillaria melea, Armillaria lutea, Oudemansiella canarii, Pycnoporus sanguineus, P. cinabarina, Tremella fuciformis, Coprinus comatus, Coprinus cinereus, Cantharellus cibarius, Hericium erinaceus, Boletus edulis, Agrocybe spp, Auricularia spp, Inocybe spp, Lactarius spp, Trametes spp, Fomes spp, Ramaria spp, Suillus spp, Collybia spp, Coriolus versicolor, Pholiota nameko, Schizophyllum commune.*

The innovation provided by the preparation of flour having nutritional value and digestibility improved by the growth of fungus mycelium means a new product and/or raw material in the market that will be of interest to consumers and industries for the development of new products.

The use of mycelium for introducing fungi into food products has been described elsewhere. Document PI0603003-3A2 discloses a process of producing flour from apple and grape bagasse myceliated with fungus of the Pleurotus genus. In spite of the similarity in object, both the substrate and the fungus used are different from the process disclosed herein, thereby distinguishing the use of the product. Document CN101095419 discloses a process for producing fungus flour.

### SUMMARY OF THE INVENTION

The present invention relates to the production of flours from grains myceliated with different types of fungi, namely, basidiomycetes and ascomycetes, having known nutritional and medicinal properties, either in admixture or not, so as to provide a product having high nutritional value.

The invention relates to a process for producing and preparing flour, which begins with the preparation of fungi and grains and ends with milling of the grains.

The present disclosure also refers to the myceliated grain flour, which can be prepared from different grains, either germinated (sprouts) or not, being preferably: wheat, triticale, sorghum, glutinous sorghum, corn, maize, amaranth, coffee, sesame, linseed, quinoa, barley, rye, rice, glutinous rice, beans, sunflower, peanut, pea, lentil, chickpea, millet, oat, soy and the like, either in admixture or not, myceliated with different edible and/or medicinal mushroom species, preferably: *Pleurotus ostreatus, P. sajor-caju, P*. *ostreatoroseus, P. eringii, Ganoderma lucidum, G. Applanatum, Ganoderma tsugae, Flammulina velutipes, Lentinula edodes, Lentinus strigellus, Morchella esculenta, M. conica, Macrolepiota procera, Volvariella volvacea, Grifola frondosa, Agaricus bisporus, A. blazei* or *A. brasiliensis, A. bitorques, A. brunnensis, Armillaria melea, Armillaria lutea, Oudemansiella canarii, Pycnoporus sanguineus, P. cinabarina, Tremella fuciformis, Coprinus comatus, Coprinus cinereus, Cantharellus cibarius, Hericium erinaceus, Boletus edulis, Agrocybe spp, Auricularia spp, Inocybe spp, Lactarius spp, Trametes spp, Fomes spp, Ramaria spp, Suillus spp, Collybia spp, Coriolus versicolor, Pholiota nameko, Schizophyllum commune.*

### BRIEF DESCRIPTION OF THE FIGURE

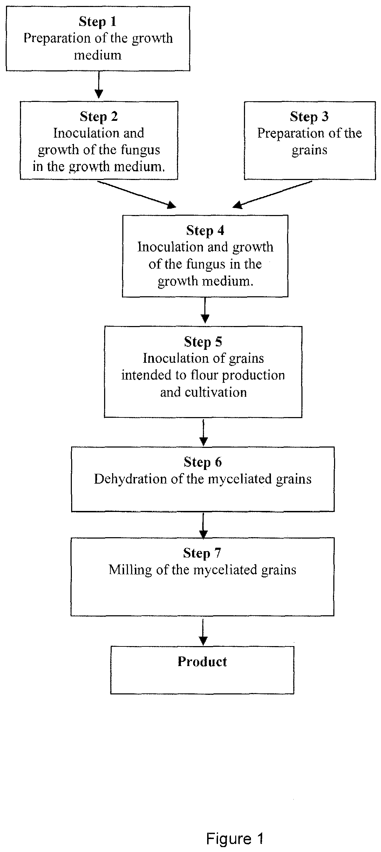

**Figure 1****.** Process for the production of flour from fungus-myceliated grains, from the preparation of the grains and fungi to the attainment of the end product.

### DETAILED DESCRIPTION OF THE INVENTION

The present invention relates to a method for making flour having grains myceliated with different fungi, preferably basidiomycetes and ascomycetes. This process adds more nutritive value to flours, making them rich in several components of the human and animal diet, thereby reducing the cost of manufacturing and the time spent with the process as compared with current processes. These flours may be used for domestic and industrial preparation/manufacture of food such as vitamins, porridges, yogurts, soups, breads, biscuits, cakes, doughs, energy bars, cereals, fodder, and the like. The method can also be used to obtain active ingredients (ergosterol, beta glucan, linoleic and oleic acid, lectins, and the like), enzymes, proteins, amino acids, vitamins, mineral salts and the like for use in the chemical, foodstuff, cosmetics, phytotherapeutic, pharmaceuticals, textiles, paper and medicine industries.

The method proposed herein comprises the following steps:
**1 - Preparation of the culture medium:** Preparing PDA culture medium (potato-dextrose-agar) which provides the initial growth of the desired fungus previously isolated. The mode of preparation consists of: boiling 100 to 200 g of potato in 500 mL of water until the consistency is reduced; filtering the content through gauze to obtain the liquid; adding 10 to 30 g agar and 10 to 30 g dextrose to the liquid at a high temperature; homogenizing the mixture and adding distilled water q.s. to make 1,000 mL. For better results, we recommend using 200 g potato, 15 g agar and 17 g dextrose. Alternatively, commercial PDA medium may be used. In case of preparation of different volumes, ratios should be respected; distribute the medium in smaller autoclavable bottles, preferably, in 250 mL Erlenmeyer flasks, seal them properly using, preferably, hydrophobic cotton plugs covered with a piece of Kraft paper and aluminum foil tied to the mouth of the container with a string; then, sterilize the material by autoclaving at 115 to 130°C for 20 to 30 minutes. For better results, we recommend autoclaving at 121°C for 30 minutes. Other solid, semi-solid and/or liquid culture media commonly used for cultivation of fungus may be used, but PDA is the most suitable. After sterilization, the medium should be placed in an aseptic environment and aliquoted in suitable sterile containers, pre-autoclaved Petri dishes being recommended; the container is allowed to rest until solidification of the medium, if a solid or semi-solid medium is used; the entire procedure should be carried out in a sterile environment, using sterilized tools and apparatuses with due care to prevent contamination of the culture, the grains and the product by undesirable microorganisms. Asepsis should be maintained until the end of step 5;
**2 - Inoculation and growth of the fungus in the growth medium:** With the aid of a suitable sterile apparatus, we recommend using a platinum or nickel-chromium wire loop or pincers, excise a small fragment (about 1 or 2 mm) of the mycelium of the fungus of interest isolated from the stock culture and seed it in a flask containing the culture medium; spread the inoculum over the medium taking care not to disrupt the intermediate layer; the streaking technique is recommended to spread the inoculum. After inoculation, the flask containing the fungus should be placed in an environment with a temperature controlled according to the optimum temperature for growth of the selected fungus; B.OD. is recommended for growth at temperatures of about 15 to 35 °C for 15 to 40 days. For better results, we recommend using a temperature of about 25 °C for 30 days of culture;
**3 - Preparation of the grains:** The selected grains should be washed and soaked in water for 3 to 15 hours, 12 hours of soaking in a volume of water equal to 3 times the grains volume being recommended; alternatively, the selected grains may be boiled in water for about 10 to 20 minutes. For better results we recommend boiling for 15 minutes and then washing; regardless of which process is used, the excess water should be removed from the grains, while keeping the moisture needed for the next step of sterilization treatment. Then the wet grains are subjected to sterilization to kill undesirable microorganisms; autoclaving under conditions similar to those described above is recommended and pasteurization, chemical or physical treatment may also be used to sterilize the grains; then, the grains are placed in plastic bags to 3/4 of the volume or trays. In this particular step, calcium carbonate or agricultural plaster should not be added, since the final purpose is not to produce "mushroom spawn" but to use the grains for food and other purposes, which is the main object of the present invention;
**4 - Inoculation of the matrix grains and growth:** Next, in order to obtain matrix grains, the grains should be inoculated with about 0.5 to 2 cm² of the fungal mycelium grown in the culture medium (step 2) with the aid of a suitable sterile apparatus. In this case the use of a platinum or nickel-chromium wire loop or pincers in a sterile environment is recommended. The bags or trays are then closed and placed in a suitable location for incubation at temperatures of about 15 to 35°C for 15 to 40 days. For better results, we recommend using a temperature of about 25°C or according with the needs of each fungus species, and then the grains will be colonized by the fungus within a period of about 30 days;
**5 - Inoculation of grains intended to flour production and cultivation:** Next, the grains intended to flour production *per se* should be inoculated with the fungal mycelium grown in the matrix grain, being thereafter placed in a location having temperature, light, aeration and moisture set according to the needs of the fungus species inoculated for about 15 to 40 days. For better results, we recommend using temperatures of 25°C for 30 days.
**6 - Dehydration of the myceliated grains:** After the incubation period, about 15 to 40 days, the myceliated grains should be placed in dehydrators with continuous air flow and a temperature of about 45 to 70°C for two days or until they are completely dry. For better results, we recommend a drying temperature of 60°C.
**7 - Milling of the myceliated grains:** After drying, the myceliated grains are milled, preferably using suitable mills for milling food or disintegrators, and then transformed into flour from whole grains or part of the grain to increase the mycelium concentration in the final flour product, which is then stored or used as raw material. The flour obtained after milling can be mixed manually or by means of conventional mixing machines to one or more flours, either myceliated or not, thereby providing a new product.

After step 3 there is no addition of calcium carbonate, plaster or any other chemical material to the grains to facilitate mycelium growth, which distinguishes the process described herein from the usual processes of producing "mushroom spawn". In addition, the "myceliated grains" used only as an inoculum to substrates or compounds for the production of the fungus are processed according to a new approach that includes the steps of dehydration and disintegration of the myceliated grains for different uses.

The flour may be produced from different grains, either in admixture or not: wheat, triticale, sorghum, corn, maize, amaranth, quinoa, coffee, sesame, linseed, rye, rice, sunflower, peanut, pea, lentil, chickpea, millet, oat, soy, and the like. Such grains may be used in the germinated form, i.e., as sprouts, or not. Many different fungus species may be used: *Pleurotus ostreatus, P. sajor-caju, P. ostreatoroseus, P. eringii, Ganoderma lucidum, G. applanatum, Ganoderma tsugae, Flammulina velutipes, Lentinula edodes, Lentinus strigellus, Morchella esculenta, M. conica, Macrolepiota procera, Volvariella volvacea, Grifola frondosa, Agaricus bisporus, A. blazei ou A. brasiliensis, A. bitorques, A. brunnensis, Armillaria melea, Armillaria lutea, Oudemansiella canarii, Pycnoporus sanguineus, P. cinabarina, Tremella fuciformis, Coprinus comatus, Coprinus cinereus, Cantharellus cibarius, Hericium erinaceus, Boletus edulis, Agrocybe spp, Auricularia spp, Inocybe spp, Lactarius spp, Trametes spp, Fomes spp, Ramaria spp, Suillus spp, Collybia spp, Coriolus versicolor, Pholiota nameko, Schizophyllum commune,* either in admixture or not.

### EXAMPLE

The invention will now be described in greater detail by means of an example, which should not be construed as a limitation on the scope of the invention, but as the best mode to understand the method and the products obtained therefrom.

Example 1: Emperor's flour (Farinha do Imperador) - flour produced from myceliated sorghum grains and the fungus *Ganoderma lucidum* for direct use in feeding.

Emperor's flour is related to the segment of food products and may be added to vitamins, porridges, yogurts, soups and the like or may be used as a raw material in the preparation of elaborate foods such as breads, biscuits, cakes, doughs, energy bars, etc. In addition to the enriched nutritional value, consumption of this flour may provide improved health. Scientific reports have shown that the fungus *Ganoderma lucidum* is a powerful ally in the treatment of several immune-related diseases, cancer and high cholesterol.

Fungi are considered a relatively expensive product to be added to basic foods, but when produced in large scale, as in the method for producing Emperor's flour, these values are sufficiently reduced to make viable their use in a competitive market, to make their nutritional values available and to allow the addition of fungi to elaborate foods. This fact also influences in a positive manner the interest of food industries in the development of new products.

Nutritional and medicinal values of Emperor's flour may bring a revolution in the field of food products, since the search for high-quality proteins derived from fungi and the great production capability have been a hope to solve the supply of nutritive foods in the growing world population, table 1. This is mainly because this process of production provides a protein-enriched product, table 2.

**Table 1 - Nutrition facts of Emperor's flour - flour produced using the method for the preparation of sorghum grains myceliated with fungus Ganoderma lucidum.**

| Amount | /100g | /30g | %DV |
|---|---|---|---|
| Calories (kcal) | 338 | 101 | 5 |
| Carbohydrates (g) | 70.3 | 21.1 | 7 |
| Proteins (Nx5,75) (g) | 7.6 | 2.28 | 3 |
| Total fat (g) | 2.9 | 0.87 | 2 |
| Saturated | 0.57 | 0.17 | - |
| Unsaturated | 2.21 | 0.67 | - |
| Trans | <0.01 | <0.01 | - |
| Cholesterol (mg) | ND | - | - |
| Total dietary fiber (g) | 12.11 | 3.63 | 15 |
| Calcium (mg) | 8.50 | 2.55 | 0 |
| Iron (mg) | 1.88 | 0.56 | 4 |
| Sodium (mg) | 0.269 | 0.08 | 0 |

| | | | |
|---|---|---|---|
| Source: Analysis performed at ITAL | | | |

**Table 2 - Protein levels of flours obtained from the method for the production of sorghum grains myceliated with five different types of fungi incubated for 30 days as compared with sorghum flour with no fungus added.**

| Material analyzed | Sorghum Protein (%) |
|---|---|
| Sorghum with no mushroom | 13.11 |
| Sorghum with *Ganoderma lucidum* | 13.99 |
| Sorghum with *Lentinula edodes* | 14.89 |
| Sorghum with *Pleurotus ostreatus* | 16.38 |
| Sorghum with *Lentinus strigellus* | 16.96 |
| Sorghum with *Agaricus blazei* | 19.21 |

Emperor's flour, whose nutritional value and digestibility were improved by the addition of the fungus *Ganoderma lucidum*, contains 52.29% beta-glucan (β-1,3 and β-1,6), which value is higher than other grains such as oat and barley that contain from 4 to 9%. It is rich in fiber, comprising 12.11% total dietary fiber, 0.21% of them being soluble fibers and 11.90% being insoluble fibers, table 3.

**Table 3 - Nutritional facts of Emperor's flour - flour produced using the method for the preparation of sorghum grains myceliated with fungus Ganoderma lucidum.**

| Amount | /100g | /30g | %DV |
|---|---|---|---|
| Moisture (g) | 6.0 | 1.80 | - |
| Ash (g) | 1.1 | 0.33 | - |
| Starch (g) | 72.1 | 21.63 | - |
| Fructose (g) | NA | - | - |
| Glucose (g) | 1.6 | 0.48 | - |
| Sucrose (g) | NA | - | - |
| Soluble dietary fiber (g) | 0.21 | 0.06 | - |
| Insoluble dietary fiber (g) | 11.90 | 3.57 | - |
| Non-protein nitrogen (NPN) (%) | 0.11 | 0.03 | - |
| Soluble protein (%) | 0.83 | 0.25 | - |

| | | | |
|---|---|---|---|
| Source: Analysis performed at ITAL | | | |

Emperor's flour contains no cholesterol and has 2.9% total fats, which is a relatively low level. However, it should be noted that 80% of this fat is comprised of unsaturated fatty acids, mainly, 1.08g/100g linoleic acid (omega 6) and 1.08 g/100 g oleic acid (omega 9), which is more desirable for animal consumption. It also contains 0.04 g/100 g omega 3. This is a favorable nutritional characteristic since unsaturated fatty acids are essential in the animal diet and saturated fatty acids can be detrimental to health. It should also be noted that the high percentage of unsaturated fatty acids is mainly caused by linoleic and oleic acids, which are important factors for Emperor's flour to be considered a healthy food, tables 4 and 5.

**Table 4 - Fatty acids present in Emperor's flour- flour produced using the method for the preparation of sorghum grains myceliated with fungus Ganoderma lucidum.**

| Fatty acids (g) | /100g | /30g | %DV |
|---|---|---|---|
| Saturated | 0.57 | 0.17 | 1 |
| Monounsaturated | 1.09 | 0.33 | - |
| Polyunsaturated | 1.12 | 0.34 | - |
| Omega 3 | 0.04 | 0.01 | - |
| Omega 6 | 1.08 | 0.32 | - |
| Total trans isomers | <0.01 | <0.01 | - |

| | | | |
|---|---|---|---|
| Source: Analysis performed at ITAL | | | |

**Table 5 - Fatty acids composition present in Emperor's flour- flour produced using the method for the preparation of sorghum grains myceliated with fungus Ganoderma lucidum.**

| Fatty acids composition (g) | | area % | (g/100g) | (g/30g) |
|---|---|---|---|---|
| C16:0 | palmitic | 18.0 | 0.50 | 0.15 |
| C16:1 Omega 7 | palmitoleic | 0.3 | 0.01 | <0.01 |
| C18:0 stearic | | 1.8 | 0.05 | 0.02 |
| C18:1 Omega 9 | trans elaidic | 0.1 | <0.01 | <0.01 |
| C18:1 Omega 9 | oleic | 39.0 | 1.08 | 0.32 |
| C18:2 Omega 6 | linoleic | 38.8 | 1.08 | 0.32 |
| C18:3 Omega 3 | alpha linolenic | 1.3 | 0.04 | 0.01 |
| C20:0 | arachidic | 0.2 | 0.01 | <0.01 |
| C20:1 Omega 11 | cis-11-eicosenoic | 0.1 | <0.01 | <0.01 |
| C22:0 | behenic | 0.1 | <0.01 | <0.01 |
| C24:0 | lignoceric | 0.3 | 0.01 | <0.01 |

| | | | | |
|---|---|---|---|---|
| Source: Analysis performed at ITAL | | | | |

Another advantage of Emperor's flour is the diversity in the amino acid composition of the proteins present therein, containing 19 different types of amino acids, having high levels of leucine in addition to considerable levels of arginine, tryptophan, lysine and methionine, which are needed for a perfect nutrition and are absent or present in small amounts in grains in general, table 6. This fact makes Emperor's flour richly supplemented with a wide variety of essential nutrients in the animal diet, table 7.

**Table 6 - Amino acids present in Emperor's flour- flour produced using the method for the preparation of sorghum grains myceliated with fungus Ganoderma lucidum.**

| Total amino acids(mg) | /100g | /30g | % DV |
|---|---|---|---|
| Glutamic Acid | 2121.87 | 636.56 | - |
| Leucine | 1241.95 | 372.59 | - |
| Alanine | 943.35 | 283.01 | - |
| Proline | 701.69 | 210.51 | - |
| Aspartic Acid | 620.93 | 186.28 | - |
| Phenylalanine | 438.79 | 131.64 | - |
| Serine | 425.56 | 127.67 | - |
| Valine | 385.32 | 115.60 | - |
| Isoleucine | 359.97 | 107.99 | - |
| Ammonia | 305.86 | 91.76 | - |
| Threonine | 301.59 | 90.48 | - |
| Glycine | 279.37 | 83.81 | - |
| Arginine | 271.08 | 81.32 | - |
| Tyrosine | 242.29 | 72.69 | - |
| Tryptophan | 198.61 | 59.58 | - |
| Histidine | 161.25 | 48.38 | - |
| Lysine | 157.61 | 47.28 | - |
| Methionine | 92.95 | 27.89 | - |
| Cystine | 18.23 | 5.47 | - |

| | | | |
|---|---|---|---|
| Source: Analysis performed at the ITAL | | | |

**Table 7 - Presentation and properties of Emperor's flour- flour produced using the method for the preparation of sorghum grains myceliated with fungus Ganoderma lucidum.**

| Cereal technology - CERES | |
|---|---|
| Beta-glucans (β-1,3 and β-1,6) (g) | 52.29 g/100g |

| Physicochemical | |
|---|---|
| Ascorbic acid (vit. C) | 15.23 mg/100g |

| Sensory | |
|---|---|
| Aspect | Fine and homogeneous powder |
| Color | Light brown |
| Odor | Characteristic |
| Flavor | Characteristic |

| | |
|---|---|
| Source: Analysis performed at LABCAL | |

### Example 2: Wheat flour with Ganoderma lucidum mycelium - flour produced from myceliated wheat grains and the fungus Ganoderma lucidum.

Wheat flour with *Ganoderma lucidum* has higher costs than Emperor's flour. Nevertheless, it has a wide use in processed foods as it is made from wheat grain and, when compared with the costs of the fungus, the use thereof is viable.

Wheat flour with *Ganoderma lucidum*, whose nutritional value and digestibility was improved by the addition of the fungus *Ganoderma lucidum*, is rich in fiber, containing 11% total dietary fiber, 0.70% being soluble fibers and 10.30% being insoluble fibers. It contains gluten.

Wheat flour with *Ganoderma lucidum*contains no cholesterol and has 2.6% total fats, which is a relatively low level. However, it should be noted that 61.5% of this fat is comprised of unsaturated fatty acids, mainly 1.17g/100g linoleic acid (omega 6) 0.36/100g oleic acid (omega 9) and 0.06/100g omega 3, which is more desirable for consumption.

Another advantage of wheat flour with Ganoderma lucidum is the diversity in the amino acids composition of the proteins present therein, containing 19 different types of amino acids, having high levels of leucine in addition to considerable levels of lysine, arginine, tryptophan and methionine, which are needed for a perfect nutrition and are absent or present in small amounts in grains in general.

Example 3: Blazei Brazil Imperial Biscuits (Biscoitos do Imperador Blazei Brazil) - biscuit produced from sorghum enriched with mycelium mushroom of the legendary king.

The Imperial Cookies Blazei Brazil are a tasty example of the potential from the innovative Emperor's Flour produced from sorghum enriched with mycelium mushroom of the legendary king. It was idealized from the consumption of the legendary king by Chinese and Japanese, who considered it the "herb of longevity", and from its nutritional and medicinal properties. This mushroom was preferably consumed by Chinese emperors because of food properties and rareness.

The Imperial Cookies are a sweet and tasty choice for those who have busy lives and seek convenience with balanced and nutritious diet. It is ideal for snacks at any time of day. The ingredients of Imperial Cookies are: tapioca flour, wheat flour, Emperor's flour, vegetable fat, cheese, eggs, coconut, sugar and salt. It also contains beta-glucan (β-1,3 and β-1,6) = 36.41g/100g.

### Method of preparation: Flour production.

The preparation of wheat and sorghum flour enriched with fungus mycelium was made in accordance with the following steps. It should also be noted that in phase III, grains were not boiled, but soaked, which results in savings in production costs and non-use of the project funds intended to gas purchase.
**Step 1 - Preparation of the growth medium** - Basic medium, maltodextrin and PDA culture media were tested. However, PDA (potato-dextrose-agar) culture medium was the most suitable. Said medium is prepared as follows: boiling 200 g of potato in 500 mL water until consistency is reduced; filtering through gauze to obtain the liquid; adding 15 g agar and 17 g dextrose to the hot liquid, stir well and bring the volume to 1000 mL with distilled water; distributing the dissolved medium into Pyrex flasks of about 250 mL, half cover the flasks and sterilize them in an autoclave at 121°C for 30 minutes. After autoclaving is complete, the medium is placed in an aseptic environment (laminar flow chamber) for the medium to cool enough to be tolerated by hand. Then the medium is transferred to Petri dishes previously sterilized by flaming the mouth of the flask in the flame of an alcohol lamp and the medium solidifies within 10 minutes.
**Step 2 - Inoculation of the fungus in the growth medium:** - A small fragment (about 1 or 2 mm) from the inner part of a healthy mushroom was removed with a pincer and placed on the growth medium in the center of the Petri dish. This operation was made carefully near the flame of the alcohol lamp inside a laminar flow chamber to prevent the powder and/or microorganisms in the air from contaminating the growth medium. After inoculation, the plates were placed in a room at a temperature of about 25°C and were kept there for about 15 days for the fungal mycelium to grow.
**Step 3 - Preparation of the grains -** wheat or sorghum grains were soaked in 3 times their volume of water for 12 hours. After the excess water was drained, the grains were placed in high-density polypropylene plastic bags to about 3/4 of the volume and were sterilized in an autoclave for 20 minutes. After sterilization, the grains were placed in a laminar flow chamber to cool down. In phase III, the boiling method was not used since the soaked grains provided satisfactory results, reducing the production costs thereby preventing the use of gas for heating.
**Step 4 - Inoculation and growth of the fungus in the growth medium** Inside a laminar flow chamber, the grains were inoculated with about 1 cm² of the fungal mycelium grown on the growth medium with the aid of a pincer and near the flame. The bags were tied with a coated wire and placed in a location at 25°C (incubation). They were colonized by the fungus within 30 days after incubation.
**Step 5 - Inoculation of grains intended to flour production and cultivation** - In the laminar flow chamber, the grains were inoculated with the mushroom mycelium grown in the matrix grain, being then placed in a location at 25°C (incubation) and after 30 days the grains were colonized by the fungus.
**Step 6 - Dehydration of the myceliated grains** - After 30 days of incubation, the grains with the mushroom mycelium were placed in dehydrators with continuous air flow and a temperature of about 60°C for two days or until they were completely dry.
**Step 7 - Milling of the myceliated grains** - The dried grains were milled in an equipment designed to flour production, resulting in a product ready to be packed.

### Nutritional analysis of the products

First, the basic composition of the different grains myceliated with different mushrooms was analyzed to provide information to serve as a basis for choosing the best combinations for a more detailed analysis. Beta-glucan levels were analyzed first since it is the main active component of fungi. The results are presented below in table 8:

**Table 8 - Beta-glucan levels of flours produced from different fungus myceliated grains.**

| Material | Beta-gl ucan level (β-1,3 and β-1,6) |
|---|---|
| *Ganoderma lucidum* mycelium | 37.27g/100g |
| Sorghum flour with *Ganoderma lucidum* | 52.29g/100g |
| Sorghum flour with *Tremela fuciformis* | 50.98g/100g |
| Sorghum flour with *Lentinula edodes* | 50.52g/100g |
| Sorghum flour with *Pleurotus ostreatus* | 33.62g/100g |
| Rice flour with *Flammulina velutipes* | 65.83g/100g |
| Rice flour with *Ganoderma lucidum* | 61.96g/100g |
| Corn flour with *Lentinula edodes* | 47.95g/100g |
| Corn flour with *Ganoderma lucidum* | 47.80g/100g |
| Millet flour with *Ganoderma lucidum* | 29.08g/100g |
| Sunflower flour with *Ganoderma lucidum* | 4.17g/100g |

Sorghum myceliated with *Ganoderma lucidum* was chosen for having substantial beta-glucan content and since the process for the production thereof is safer in relation to contamination risks than that used for rice.

Then, analyses were performed at the prestigious Institute of Food Technology (Instituto de Tecnologia de Alimentos - ITAL) and at the prestigious Analysis Laboratory - LABCAL of the Federal University of Santa Catarina (Laboratório de Análises - LABCAL da Universidade Federal de Santa Catarina) for sorghum and wheat flour with *Ganoderma lucidum* and the mycelium of this mushroom. Results are presented below in tables 9 to 14:

**Table 9 - Comparison of the analysis of nutritional facts of mycelium and flours from sorghum and wheat with Ganoderma lucidum .**

| Amount | Sorghum flour with *G. lucidum* | | | Wheat flour with *G. lucidum* | | | Mycelium of *G. lucidum* | | |
|---|---|---|---|---|---|---|---|---|---|
| | /100g | /30g | %DV | /100g | /30g | %DV | /100g | /30g | %DV |
| Calories (kcal) | 338 | 101 | 5 | 332 | 100 | 5 | 176 | 53 | 3 |
| Carbohydrates (g) | 70.3 | 21.1 | 7 | 64.5 | 19.4 | 6 | 24.4 | 7.3 | 2 |
| Proteins (Nx5,75) (g) | 7.6 | 2.28 | 3 | 12.7 | 3.8 | 5 | 12.5 | 3.8 | 5 |
| Total fat (g) | 2.9 | 0.87 | 2 | 2.6 | 0.8 | 1 | 3.1 | 0.9 | 2 |
| Saturated | 0.57 | 0.17 | - | 0.52 | 0.16 | 1 | 0.52 | 0.16 | 1 |
| Unsaturated | 2.21 | 0.67 | - | 1.60 | 0.48 | - | 2.02 | 0.61 | - |
| Trans | <0.01 | <0.01 | - | <0.01 | <0.01 | - | <0.01 | <0.01 | - |
| Cholesterol (mg) | ND | - | - | ND | - | - | ND | - | - |
| Total dietary fiber (g) | 12.11 | 3.63 | 15 | 11.00 | 3.30 | 13 | 46.39 | 13.92 | 56 |
| Calcium (mg) | 8.50 | 2.55 | 0 | 49.00 | 15.00 | 2 | 16.3 | 4.9 | 0 |
| Iron (mg) | 1.88 | 0.56 | 4 | 3.20 | 0.96 | 7 | 4.8 | 1.4 | 10 |
| Sodium (mg) | 0.269 | 0.08 | 0 | 0.69 | 0.21 | 0 | 0.79 | 0.24 | 0 |

| | | | | | | | | | |
|---|---|---|---|---|---|---|---|---|---|
| Source: Analysis performed at ITAL | | | | | | | | | |

**Table 10 - Comparison of the analysis of further nutritional facts of mycelium and flours from sorghum and wheat with Ganoderma lucidum .**

| | Sorghum flour with *G. lucidum* | | | Wheat flour with *G. lucidum* | | | Mycelium of *G. lucidum* | | |
|---|---|---|---|---|---|---|---|---|---|
| | /100g | /30g | %DV | /100g | /30g | %DV | /100g | /30g | %DV |
| Moisture (g) | 6.0 | 1.80 | - | 7.6 | 2.3 | - | 10.0 | 3.0 | - |
| Ash (g) | 1.1 | 0.33 | - | 1.6 | 0.5 | - | 3.7 | 1.1 | - |
| pH (10% solution) | - | - | - | 4.7 | 1.4 | - | - | - | - |
| Reducing sugars (g) | - | - | - | 1.8 | 0.5 | - | - | - | - |
| Non-reducinq sugars (g) | - | - | - | 2.2 | 0.7 | - | - | - | - |
| Total sugars (g) | - | - | - | 4.0 | 1.2 | - | - | - | - |
| Starch (g) | 72.1 | 21.63 | - | 60.9 | 18.3 | - | - | - | - |
| Fructose (g) | ND | - | - | ND | - | - | - | - | - |
| Glucose (g) | 1.6 | 0.48 | - | 1.4 | 0.4 | - | - | - | - |
| Sucrose (g) | ND | - | - | 0.6 | 0.2 | - | - | - | - |
| Ascorbic acid (mg) | ND | - | - | ND | - | - | 2.95 | 0.89 | 2 |
| Vitamin B1 - Thiamine (mg) | 0.14 | 0.04 | 4 | 0.32 | 0.10 | 8 | 0.67 | 0.20 | 17 |
| Vitamin B2 - Riboflavin (mg) | 0.14 | 0.04 | 3 | 0.11 | 0.03 | 3 | 0.95 | 0.29 | 22 |
| Niacin (mg) | 1.21 | 0.36 | 2 | 2.22 | 0.67 | 4 | 1.38 | 0.41 | 3 |
| Vitamin B6 - Pyridoxin (mg) | ND | - | - | 0.13 | 0.04 | 3 | ND | - | - |
| Alpha-tocopherol (mg) | 0.23 | 0.07 | - | 0.64 | 0.19 | - | 0.13 | 0.04 | - |
| Beta-tocopherol (mg) | ND | - | - | 0.30 | 0.09 | - | ND | - | - |
| Gamma-tocopherol (mg) | 0.42 | 0.13 | - | ND | - | - | 0.29 | 0.09 | - |
| Delta-tocopherol (mg) | ND | - | - | ND | - | - | ND | - | - |
| Total tocopherol (mq) | 0.65 | 0.20 | - | 0.93 | 0.28 | - | 0.42 | 0.13 | - |
| Vitamin E (UI) | < 1 | - | - | < 1 | - | - | < 1 | - | - |
| Vitamin E expressed as | 0.29 | 0.09 | 1 | 0.72 | 0.22 | 2 | 0.17 | 0.05 | 1 |
| alpha-tocopherol (mg) | | | | | | | | | |
| Beta-carotene (mcg) | ND | - | - | ND | - | - | ND | - | - |
| Vitamin A (UI) | ND | - | - | ND | - | - | ND | - | - |
| Total carotenoids expressed as beta-carotene (mg) | ND | - | - | ND | - | - | ND | - | - |
| Soluble dietary fiber (q) | 0.21 | 0.06 | - | 0.70 | 0.21 | - | 3.90 | 1.17 | - |
| Insoluble dietary fiber (g) | 11.90 | 3.57 | - | 10.30 | 3.09 | - | 42.49 | 12.75 | - |
| Non-protein nitrogen (NPN) (%) | 0.11 | 0.03 | - | - | - | - | - | - | - |
| Soluble protein (%) | 0.83 | 0.25 | - | - | - | - | - | - | - |
| Barium (mg) | 0.017 | 0.005 | - | 0.68 | 0.20 | - | 0.033 | 0.01 | - |
| Cobalt (mg) | ND | - | - | ND | - | - | ND | - | - |
| Copper (mg) | 0.13 | 0.04 | 0 | 0.17 | 0.05 | 0 | 0.995 | 0.299 | 0 |
| Phosphorus (mg) | 216 | 65 | 9 | 342 | 103 | 15 | 745 | 224 | 32 |
| Magnesium (mg) | 89 | 27 | 10 | 113 | 34 | 13 | 246 | 74 | 28 |
| Nickel (mg) | 0.007 | 0.002 | - | 0.003 | 0 | - | 0.018 | 0.005 | - |
| Potassium (mg) | 215 | 65 | - | 338 | 101 | - | 829 | 249 | - |
| Vanadium (mg) | ND | - | - | ND | - | - | ND | - | - |
| Zinc (mg) | 1.44 | 0.43 | 6 | 4.43 | 1.33 | 19 | 3.8 | 1.1 | 16 |

| | | | | | | | | | |
|---|---|---|---|---|---|---|---|---|---|
| Source: Analysis performed at ITAL | | | | | | | | | |

**Table 11 - Comparison of the analysis of fatty acids present in the mycelium and flours from sorghum and wheat with Ganoderma lucidum.**

| | Sorghum flour with *G. lucidum* | | | Wheat flour with *G. lucidum* | | | Mycelium of *G. lucidum* | | |
|---|---|---|---|---|---|---|---|---|---|
| | /100g | /30g | %DV | /100g | /30g | %DV | /100g | /30g | %DV |
| Saturated | 0.57 | 0.17 | 1 | 0.52 | 0.16 | 1 | 0.52 | 0.16 | 1 |
| Monounsaturated | 1.09 | 0.33 | - | 0.37 | 0.11 | - | 0.80 | 0.24 | - |
| Polyunsaturated | 1.12 | 0.34 | - | 1.23 | 0.37 | - | 1.22 | 0.37 | - |
| Omega 3 | 0.04 | 0.01 | - | 0.06 | 0.02 | - | 0.03 | 0.01 | - |
| Omega 6 | 1.08 | 0.32 | - | 1.17 | 0.35 | - | 1.19 | 0.36 | - |
| Total trans isomers | <0.01 | <0.01 | - | <0.01 | <0.01 | - | <0.01 | <0.01 | - |
| N.I | - | - | - | - | - | - | 0.02 | 0.01 | - |

| | | | | | | | | | |
|---|---|---|---|---|---|---|---|---|---|
| N.I = Not identified. Source: Analysis performed at ITAL | | | | | | | | | |

**Table 12 - Comparison of the analysis of oils present in the mycelium and flours from sorghum and wheat with Ganoderma lucidum.**

| | Sorghum flour with *G. lucidum* | | | Wheat flour with *G. lucidum* | | | Mycelium of *G. lucidum* | | |
|---|---|---|---|---|---|---|---|---|---|
| | area % | g/10 0g | g/30g | area % | g/100g | g/30g | area % | g/100 g | g/30g |
| C14:0 myristic | - | - | - | 0.2 | < 0.01 | < 0.01 | 0.3 | 0.01 | < 0.01 |
| C15:0 pentadecanoic | - | - | - | 0.2 | < 0.01 | < 0.01 | 1.1 | 0.03 | 0.01 |
| C16:0 palmitic | 18.0 | 0.50 | 0.15 | 22.8 | 0.49 | 0.15 | 15.6 | 0.40 | 0.12 |
| N.I | - | - | - | - | - | - | 0.1 | < 0.01 | < 0.01 |
| C16:1 Omega 7 palmitoleic | 0.3 | 0.01 | <0.01 | 0.1 | < 0.01 | < 0.01 | 0.4 | 0.01 | < 0.01 |
| N.I | - | - | - | - | - | - | 0.2 | 0.01 | < 0.01 |
| C17:0 margaric | - | - | - | 0.1 | < 0.01 | < 0.01 | 0.4 | 0.01 | < 0.01 |
| C18:0 stearic | 1.8 | 0.05 | 0.02 | 1.3 | 0.03 | 0.01 | 1.5 | 0.04 | 0.01 |
| C18:1 Omega 9 trans elaidic | 0.1 | <0.0 1 | <0.01 | - | - | - | - | - | - |
| C18:1 Omega 9 oleic | 39.0 | 1.08 | 0.32 | 17.1 | 0.36 | 0.11 | 30.8 | 0.78 | 0.23 |
| C18:2 Omega 6 linoleic | 38.8 | 1.08 | 0.32 | 55.0 | 1.17 | 0.35 | 46.7 | 1.19 | 0.36 |
| C18:3 Omega 3 alpha linolenic | 1.3 | 0.04 | 0.01 | 2.7 | 0.06 | 0.02 | 1.0 | 0.03 | 0.01 |
| C20:0 arachidic | 0.2 | 0.01 | <0.01 | - | - | - | 0.1 | <0.01 | <0.01 |
| C20:1 Omega 11 cis-11-eicosenoic | 0.1 | <0.0 1 | <0.01 | 0.5 | 0.01 | <0.01 | 0.3 | 0.01 | <0.01 |
| C21:0 heneicosanoic | - | - | - | - | - | - | 0.1 | <0.01 | <0.01 |
| C22:0 behenic | 0.1 | <0.0 1 | <0.01 | - | - | - | 0.1 | <0.01 | <0.01 |
| C23:0 tricosanoic | - | - | - | - | - | - | 0.2 | 0.01 | <0.01 |
| C24:0 lignoceric | 0.3 | 0.01 | <0.01 | - | - | - | 0.6 | 0.02 | 0.01 |
| N.I | - | - | - | - | - | - | 0.5 | 0.01 | <0.01 |

| | | | | | | | | | |
|---|---|---|---|---|---|---|---|---|---|
| N.I. = Not identified. Source: Analysis performed at ITAL | | | | | | | | | |

**Table 13 - Comparison of the analysis of amino acids present in the mycelium and flours from sorghum and wheat with Ganoderma lucidum.**

| | Sorghum flour with *G. lucidum* | | | Wheat flour with *G. lucidum* | | | Mycelium of *G. lucidum* | | |
|---|---|---|---|---|---|---|---|---|---|
| Amino acids (g) | /100g | /30g | % DV | /100g | /30g | % DV | /100g | /30g | % DV |
| Glutamic Acid | 2.12 | 0.64 | - | 3.89 | 1.17 | - | 1.48 | 0.44 | - |
| Leucine | 1.24 | 0.37 | - | 0.85 | 0.26 | - | 1.92 | 0.58 | - |
| Alanine | 0.94 | 0.28 | - | 0.51 | 0.15 | - | 0.81 | 0.24 | - |
| Proline | 0.70 | 0.21 | - | 1.27 | 0.38 | - | 0.51 | 0.15 | |
| Aspartic Acid | 0.62 | 0.19 | - | 0.78 | 0.23 | - | 1.02 | 0.31 | - |
| Phenylalanine | 0.44 | 0.13 | - | 0.37 | 0.11 | - | 0.42 | 0.13 | - |
| Serine | 0.43 | 0.13 | - | 0.70 | 0.21 | - | 0.56 | 0.17 | - |
| Valine | 0.38 | 0.12 | - | 0.57 | 0.17 | - | 0.51 | 0.15 | - |
| Isoleucine | 0.36 | 0.11 | - | 0.41 | 0.12 | - | 1.06 | 0.32 | - |
| Ammonia | 0.31 | 0.09 | - | 0.24 | 0.07 | - | 0.34 | 0.13 | - |
| Threonine | 0.30 | 0.09 | - | 0.41 | 0.12 | - | 0.52 | 0.16 | - |
| Glycine | 0.28 | 0.08 | - | 0.56 | 0.17 | - | 0.51 | 0.15 | - |
| Arginine | 0.27 | 0.08 | - | 0.50 | 0.15 | - | 0.43 | 0.13 | - |
| Tyrosine | 0.24 | 0.07 | - | 0.37 | 0.11 | - | 0.28 | 0.08 | - |
| Tryptophan | 0.20 | 0.06 | - | 0.26 | 0.08 | - | 0.29 | 0.09 | - |
| Histidine | 0.16 | 0.05 | - | 0.35 | 0.11 | - | 0.30 | 0.09 | - |
| Lysine | 0.16 | 0.05 | - | 0.50 | 0.15 | - | 0.54 | 0.16 | - |
| Methionine | 0.09 | 0.03 | - | 0.14 | 0.04 | - | 0.07 | 0.02 | - |
| Cystine | 0.02 | 0.01 | - | 0.07 | 0.02 | - | 0.03 | 0.01 | - |

| | | | | | | | | | |
|---|---|---|---|---|---|---|---|---|---|
| Source: Analysis performed at ITAL | | | | | | | | | |

**Table 14 - Comparison of the analysis of the contaminating microorganisms present in mycelium and flours from sorghum and wheat with Ganoderma lucidum.**

| Analyses | Sorghum flour with *G. lucidum* | Wheat flour with *G. lucidum* | Mycelium of *G. lucidum* |
|---|---|---|---|
| Cereal technology - CERES | | | |
| Beta-glucans (β-1,3 and β-1,6) (g) | 52.29 g/100g | - | 37.27 g/100g |
| Gluten detection | Not detected (limit of detection at 3 ppm) | Contains gluten (limit of detection at 3 ppm) | Not detected (limit of detection at 3 ppm) |
| Sensory | | | |
| Aspect | Fine and homogeneous powder | - | - |
| Color | Light brown | - | - |
| Odor | Characteristic | - | - |
| Flavor | Characteristic | - | - |
| Microbiology | | | |
| Molds and yeasts | 2.2 x 10⁴ UFC/g | 9.8 x 10³ UFC/g | 1.0 x 10² UFC/g |
| Coliform bacteria at 45° C | < 3 NMA/g | < 3 NMA/g | < 3 NMA/g |
| *Bacillus cereus* count | 1.0 x 10¹ UFC/g | < 1.0 x 10² UFC/g | < 1.0 x 10² UFC/g |
| Coliform bacteria count at 35° C | < 1.0 x 10¹ UFC/g | - | - |
| *Enterobacter spp* | < 3 NMA/g | < 1.0 x 10¹ UFC/g | < 1.0 x 10¹ UFC/g |
| *Escherichia coli* | < 3 NMA/g | - | - |
| *Pseudomonas spp* | < 1.0 x 10² UFC/g | - | - |
| *Salmonella spp* | Absent in 25g | - | - |
| *Staphylococcus aureus* | < 1.0 x 10¹ UFC/g | - | - |
| Mycotoxins and food contaminants | | | |
| Aflatoxins (B1, B2, G1, G2) | Not detected | Not detected | Not detected |
| Ochratoxin A | - | Not detected | Not detected |

| | | | |
|---|---|---|---|
| Source: Analysis performed at LABCAL | | | |

LABCAL informed that the test to assess the degree of gelatinization is based on the formation of a blue complex between iodine and amylase during gelatinization. However, this assessment was not possible in the sample of wheat flour with mushroom *Ganoderma lucidum* and in the mycelium, since the samples could not be read at 600 nm, possibly because of the presence of further components in addition to amylose or the low amylose content. Emperor's flour was not tested for the degree of gelatinization.

### Gastronomic test of the Emperor's flour

Based on the analyses results, the flours chosen for human consumption were tested for direct use or as a raw material in the preparation of several products. The best recipes are as follows:

### Oatmeal mush with Emperor's Flour

In a saucepan, combine 1 ½ cup of milk, 1 tablespoon of Emperor's Flour, 3 tablespoons of Fine Oat Flakes and e 1 tablespoon of brown sugar. Put the pan on high heat, stirring constantly. Let it thicken a little and add the apple while still stirring. Then, add the corn flakes and mix well. Remove from heat and serve hot with lemon zests on top.
Preparation time: 10 minutes.
Yield: 2 servings

### Apple and carrot Vitamin:

1/2 medium apple
1/2 medium carrot
2 cups of milk
1 tablespoon of Emperor's flour.
1 tablespoons wheat germ.
1 cup of orange juice.
3 tablespoons of honey.

### Prune Vitamin:

1 cup (tea) of skim milk.
1 tablespoon of Emperor's flour.
1 tablespoon of oat flakes.
1 tablespoon of honey.
4 seedless prunes.

### Strawberry vitamin :

1 tablespoon of sugar
2 tablespoons of Emperor's flour.
1 cup of clean strawberries.
1 cup of orange juice .

### Carrot, apple and orange Shake:

1 medium carrot
1 apple
2 cups of skim milk.
2 tablespoons of Emperor's flour.
1 cup of orange juice.

### Shake with cereals, nuts and flaxseed:

Two branches of mint.
1/2 cup of skim cold milk
1 tablespoon of flaxseed.
4 medium-sized nuts
4 tablespoons of Emperor's flour.
4 tablespoons of granola.
sweeten to taste.

### Imperial Biscuits

500 g sweet tapioca flour.
250 g wheat flour.
125 g Emperor's flour.
200 g grated cured minas cheese.
100 g grated coconut.
400 g refined sugar
200 ml soy oil.
100 g vegetable butter.
10 g baking powder
5 eggs.

### Preparation:

The ingredients should be at room temperature. Put all the ingredients in a bowl and knead until it can be rolled. Roll into a spiral, or any other shape. Bake in pre-heated oven at 180 degrees.

### Whole-flour Imperial Biscuits

500 g sweet tapioca flour.
250 g whole wheat flour
125 g Emperor's flour.
400 g brown sugar
200 g grated cured minas cheese.
100 g grated coconut.
200 ml vegetable oil.
100 g vegetable butter.
5 eggs.
10 g baking powder
Prepare like the first recipe.

### Corn starch biscuits (Sequilho)

700 g corn starch
150 g millet flour myceliated with *G*. *lucidum.*
150 g Emperor's flour.
300 g brown sugar.
120 g soymilk.
100 ml water to dissolve the milk.
300 g vegetable butter
200 ml coconut milk.

### Preparation:

Knead until it can be rolled. Bake in pre-heated oven at 180 degrees.

### Orange cake

3 cups of brown sugar
4 cups of whole wheat flour
1 cup of Emperor's flour.
2 cups of orange juice.
10 g baking powder
200 ml vegetable oil.

### Preparation:

Beat the ingredients and put the mix into a greased and floured cake pan. Bake in pre-heated oven at 180 to 200 degrees.

### Crispy bread

1.2 kg whole wheat flour

400 g Emperor's flour.
300 g soymilk dissolved in 450 ml of water.
40 g sesame.
1 tablespoon of salt.
20 g dried yeast.

### Preparation:

Knead until the dough leaves the sides of the bowl. Roll the dough, let it rise, then bake it.

### Imperial cereal energy bar

2 cups of dried fruits.
½ cup of honey.
4 tablespoons of orange juice.
4 tablespoons of lemon juice.
1 1/2 cups of whole wheat flour
1 cup of Emperor's flour.
½ teaspoon of sodium bicarbonate.
½ teaspoon of baking powder.
1 tablespoon of canola oil.
1/4 cup of corn syrup (karo).
2 egg whites.
1 cup of oat.
½ cup of brown sugar (optional).

### Preparation:

In a blender, beat the dried fruits, honey and orange and lemon juices. Mix the other ingredients separately and set the oats aside. Add the contents of the blender with the dough and make small flat rectangles. Sprinkle with the oat and place on a tray. Make in medium heat at 180 degrees for 15 minutes.

### Whole cereal energy bar

1 cup of white whole sesame seeds.
1 cup of fine wheat bran
1 cup of Emperor's flour.
1 cup of whole rye flakes (pre cooked).
180 g seedless raisins.
1 cup of toasted, salted and chopped cashew nuts.
2 cup of whole fine oat flakes.
2 cup of traditional brown sugar.
1 cup of water.
1 cup of honey.

### Preparation:

Boil the water, sugar and honey until strings can be formed. Put in a tray, spread it and put it on a plastic sheet and mold it.

### Economic cereal energy bar

1 cup of brown sugar.
1 tablespoon of corn syrup (karo).
1 coffea cup of water.
1 cup of wheat bran.
1 cup of Emperor's flour.
1 cup of rice flakes.
1 tablespoon of sesame seed.
3 tablespoons of gross-oats

### Preparation:

In a saucepan, add sugar, corn syrup and water. Put on heat until ball stage is reached.

In a bowl, mix the wheat bran, the oats, sesame seeds and rice flakes. Add the syrup on top of the mix and stir until a ball is formed. Put on a greased aluminum foil and press by hand until a large bar is formed with a thickness of about 1 cm. Cut smaller bars and wrap them in a plastic wrap.

It was noted that Emperor's flour may partially or completely replace wheat flour in traditional recipes and that although *Ganoderma lucidum* is not a mushroom commonly used for cooking due to the rigidity of its fruiting body, that is as rigid as wood, when in the form of a sorghum flour myceliated with *Ganoderma lucidum* it may be used in several recipes, providing nutritional components with reduced costs than dehydrated mushroom and having better taste. Imperial biscuits were distributed to people for tasting experiments in 14 natural product stores located in Brasília, and it was greatly accepted by people who tried it.

## Claims

1. Process for producing myceliated flour, **characterized in that** it comprises the following steps:
a. preparing a growth medium for fungus growth, wherein the growth medium is a potato-dextrose-agar medium prepared by using 100 to 200 g of potato, 10 to 30 g agar and 10 to 30 g dextrose; wherein step (a) is carried out in a sterile environment;
b. inoculating the fungus in the growth medium using a sterile apparatus;
c. growing in culture for 15 to 40 days at a temperature of 15 to 35°C;
d. preparing matrix grains for inoculation of fungi by: (i) soaking grains in water for 12 h, wherein the volume of water is three times the grain volume; or alternatively boiling the grains for 10 to 20 minutes, (ii) storing the grains in a suitable container to 3/4 of the volume, (iii) sterilizing the grains in a medium designed to be used with food products and (iv) cooling the grains; wherein step (d) is carried out without adding calcium carbonate or agricultural plaster;
e. inoculating the matrix grains obtained in step (d) with 0.5 to 2 cm² of the mycelium grown in the growth medium and placing them in bags or trays in a sterile environment;
f. closing the bags or trays containing the matrix grains and incubating them at a temperature between 15 and 35°C for 15 to 40 days;
g. inoculating grains intended to flour production with the fungal mycelium grown in the matrix grain as obtained in step (f);
h. incubating the inoculated grains as obtained in step (g) at a temperature between 15 and 35°C for 15 to 40 days;
i. dehydrating the myceliated grains obtained in step (h) with continuous air flow at a temperature of 60°C;
j. milling the dried grains to produce flour.

2. Process of claim 1, **characterized in that** the medium is potato-dextrose-agar solid medium.

3. Process of claim 1, **characterized in that** the fungus is selected from the group consisting of: *Flammulina velutipes, Pleurotus ostreatus, P. sajor-caju, P. ostreatoroseus, P. eringii*, *Ganoderma lucidum, G. Applanatum, Ganoderma tsugae, Flammulina velutipes, Lentinula edodes, Lentinus strigellus, Morchella esculenta, M. conica, Macrolepiota procera, Volvariella volvacea, Grifola frondosa, Agaricus bisporus, A. blazei or A. brasiliensis, A. bitorques, A. brunnensis, Armillaria melea, Armillaria lutea, Oudemansiella canarii, Pycnoporus sanguineus, P. cinabarina, Tremella fuciformis, Coprinus comatus, Coprinus cinereus, Cantharellus cibarius, Hericium erinaceus, Boletus edulis, Agrocybe spp, Auricularia spp, Inocybe spp, Lactarius spp, Trametes spp, Fomes spp, Ramaria spp, Suillus spp, Collybia spp, Coriolus versicolor, Pholiota nameko, Schizophyllum commune*, or different edible and/or medicinal fungus species.

4. Process of claim 1, **characterized in that** the inoculation of the grains of step (e) is performed with 1 cm² of the mycelium.

5. Process of claim 1, **characterized in that** the grains are selected from the group consisting of: wheat, triticale, sorghum, glutinous sorghum, corn, maize, amaranth, coffee, sesame, linseed, quinoa, barley, rye, rice, glutinous rice, beans, sunflower, peanut, pea, lentil, chickpea, millet, oat, and soy.

6. Process of claim 1, **characterized in that** the bag or tray is a polypropylene plastic bag or tray.

## Patentansprüche

1. Verfahren zur Herstellung von myzeliertem Mehl, **dadurch gekennzeichnet, dass** es die folgenden Schritte umfasst:
a. Herstellen eines Wachstumsmediums für Pilzwachstum, wobei das Wachstumsmedium ein Medium aus Kartoffel, Dextrose und Agar ist, das unter Verwendung von 100 bis 200 g Kartoffel, 10 bis 30 g Agar und 10 bis 30 g Dextrose hergestellt wurde; wobei Schritt (a) in einer sterilen Umgebung durchgeführt wird;
b. Inokulation des Pilzes in das Wachstumsmedium unter Verwendung eines sterilen Geräts;
c. in Kultur wachsen lassen für 15 bis 40 Tage bei einer Temperatur von 15 bis 35°C;
d. Herstellen von Matrixkörnern zur Inokulation von Pilzen durch: (i) Einweichen von Körnern in Wasser für 12 Stunden, wobei das Volumen von Wasser dreimal so groß ist wie das Kornvolumen; oder alternativ Kochen der Körner für 10 bis 20 Minuten, (ii) Lagern der Körner in einem geeigneten Behälter bis 3/4 des Volumens, (iii) Sterilisieren der Körner in einem Medium, das dazu vorgesehen ist, mit Nahrungsmittelprodukten verwendet zu werden, und (iv) Kühlen der Körner; wobei Schritt (d) ausgeführt wird, ohne dass Calciumcarbonat oder Agrargips hinzugefügt wird;
e. Inokulation der Matrixkörner, die in Schritt (d) erhalten wurden, mit 0,5 bis 2 cm² des in dem Wachstumsmedium gewachsenen Myzeliums und Abfüllen in Säcke oder Trays, in einer sterilen Umgebung;
f. Schließen der Säcke oder Trays, die die Matrixkörner enthalten, und Inkubieren derselben bei einer Temperatur zwischen 15 und 35°C für 15 bis 40 Tage;
g. Inokulation der Körner, die für die Mehlproduktion bestimmt sind, mit dem Pilzmyzelium, das in dem in Schritt (f) erhaltenen Matrixkorn wachsen gelassen wurde;
h. Inkubieren der inokulierten Körner, die in Schritt (g) erhalten wurden, bei einer Temperatur zwischen 15 und 35°C für 15 bis 40 Tage;
i. Dehydratisieren der myzelierten Körner, die in Schritt (h) erhalten wurden, mittels eines kontinuierlichen Luftstroms bei einer Temperatur von 60°C;
j. Mahlen der getrockneten Körner, um Mehl herzustellen.

2. Verfahren nach Anspruch 1, **dadurch gekennzeichnet, dass** das Medium ein festes Medium aus Kartoffel, Dextrose und Agar ist.

3. Verfahren nach Anspruch 1, **dadurch gekennzeichnet, dass** der Pilz ausgewählt ist aus der Gruppe bestehend aus: *Flammulina velulipes, Pleurotus oslreatus, P. sajor-caju, P. ostreatoroseus, P. eringii, Ganoderma lucidum, G. Applanatum, Ganoderma Isugae, Flammulina velulipes, Lenlinula edodes, Lentinus strigellus, Morchella esculenta, M, conica, Macrolepiota procera, Volvariella volvacea, Grifola frondosa, Agaricus bisporus, A. blazei oder A. brasiliensis, A. bitorques, A. brunnensis, Armillaria melea, Annillaria lutea, Oudemansiella canarii, Pycnoporus sanguineus, P. cinabarina, Tremella fuciformis, Coprinus comatus, Coprinus cinereus, Cantharellus cibarius, Hericium erinaceus, Boletus edulis, Agrocybe spp, Auricularia spp, Inocybe spp, Lactarius spp, Trametes spp, Fomes spp, Ramaria spp, Suillus spp, Collybia spp, Coriolus versicolor, Pho*/*iota nameko, Schizophyllum commune,* oder verschiedenen essbaren und/oder medizinischen Pilzarten.

4. Verfahren nach Anspruch 1, **dadurch gekennzeichnet, dass** die Inokulation der Körner in Schritt (e) mit 1 cm² des Myzeliums durchgeführt wird.

5. Verfahren nach Anspruch 1, **dadurch gekennzeichnet, dass** die Körner ausgewählt sind aus der Gruppe bestehend aus: Weizen, Triticale, Sorghum, Klebsorghum, Getreide, Mais, Amarant, Kaffee, Sesam, Leinsamen, Quinoa, Gerste, Roggen, Reis, Klebreis, Bohnen, Sonnenblumen, Erdnüssen, Erbsen, Linsen, Kichererbsen, Hirse, Hafer und Soja.

6. Verfahren nach Anspruch 1, **dadurch gekennzeichnet, dass** der Sack oder das Tray ein Sack oder Tray aus Polypropylenplastik ist.

## Revendications

1. Procédé de production de farine mycéliée, **caractérisé en ce qu'**il comprend les étapes suivantes :
a. préparation d'un milieu de croissance pour la croissance de champignon, le milieu de croissance étant un milieu de pomme de terre-dextrose-agar préparé en utilisant 100 à 200 g de pommes de terre, 10 à 30 g d'agar et 10 à 30 g de dextrose ; l'étape (a) étant réalisée dans un environnement stérile ;
b. inoculation du champignon dans le milieu de croissance en utilisant un appareil stérile ;
c. croissance dans une culture pendant 15 à 40 jours à une température de 15 à 35°C ;
d. préparation des grains de matrice pour l'inoculation des champignons en : (i) plongeant les grains dans de l'eau pendant 12 h, le volume d'eau étant de trois fois le volume des grains ; ou alternativement, en faisant bouillir les grains pendant 10 à 20 minutes, (ii) stockant les grains dans un récipient approprié à 3/4 du volume, (iii) stérilisant les grains dans un milieu conçu pour être utilisé avec des produits alimentaires et (iv) refroidissant les grains ; l'étape (d) étant réalisée sans ajouter de carbonate de calcium ou de plâtre agricole ;
e. inoculation des grains de matrice obtenus à l'étape (d) avec 0,5 à 2 cm² du mycélium cultivé dans le milieu de croissance et leur placement dans des sacs ou bacs dans un environnement stérile ;
f. fermeture des sacs ou bacs contenant les grains de matrice et leur incubation à une température comprise entre 15 et 35°C pendant 15 à 40 jours ;
g. inoculation des grains destinés à la production de farine avec le mycélium fongique cultivé dans le grain de matrice tel qu'obtenu à l'étape (f) ;
h. incubation des grains inoculés tels qu'obtenus à l'étape (g) à une température comprise entre 15 et 35°C pendant 15 à 40 jours ;
i. déshydratation des grains mycéliés obtenus à l'étape (h) avec un flux d'air continu à une température de 60°C ;
j. broyage des grains séchés pour obtenir de la farine.

2. Procédé selon la revendication 1, **caractérisé en ce que** le milieu est un milieu solide de pomme de terre-dextrose-agar.

3. Procédé selon la revendication 1, **caractérisé en ce que** le champignon est choisi dans le groupe constitué de : *Flammulina velutipes, Pleurotus ostreatus, P. sajor-caju, P. ostreatoroseus, P. eringii, Ganoderma lucidum, G. Applanatum, Ganoderma tsugae, Flammulina velutipes, Lentinula edodes, Lentinus strigellus, Morchella esculenta, M. conica, Macrolepiota procera, Volvariella volvacea, Grifola frondosa, Agaricus bisporus, A. blazei* ou *A. brasiliensis, A. bitorques, A. brunnensis, Armillaria melea, Armillaria lutea, Oudemansiella canarii, Pycnoporus sanguineus, P. cinabarina, Tremella fuciformis, Coprinus comalus, Coprinus cinereus, Cantharellus cibarius, Hericium erinaceus, Boletus edulis, Agrocybe spp, Auricularia spp, Inocybe spp, Lactarius spp, Trametes spp, Fomes spp, Ramaria spp, Suillus spp, Collybia spp, Coriolus versicolor, Pholiota nameko, Schizophyllum commune* ou différentes espèces de champignon comestibles et/ou médicinales.

4. Procédé selon la revendication 1, **caractérisé en ce que** l'inoculation des grains de l'étape (e) est réalisée avec 1 cm² de mycélium.

5. Procédé selon la revendication 1, **caractérisé en ce que** les grains sont sélectionnés dans le groupe constitué de : blé, triticale, sorgho, sorgho gluant, céréale, maïs, amarante, café, sésame, graines de lin, quinoa, orge, seigle, riz, riz gluant, haricots, tournesol, arachide, pois, lentilles, pois chiche, millet, avoine et soja.

6. Procédé selon la revendication 1, **caractérisé en ce que** le sac ou bac est un sac ou bac en plastique de polypropylène.
